(19) Europäisches Patentamt
European Patent Office
Office européen des brevets

(11) Veröffentlichungsnummer: **0 092 738**
**B1**

# (12) EUROPÄISCHE PATENTSCHRIFT

(45) Veröffentlichungstag der Patentschrift: **24.07.85**

(51) Int. Cl.[4]: **C 07 C 119/042**, C 07 C 119/045, C 07 C 119/048, C 07 C 118/00

(21) Anmeldenummer: **83103587.8**

(22) Anmeldetag: **14.04.83**

## (54) Verfahren zur kontinuierlichen thermischen Spaltung von Carbamidsäureestern.

(30) Priorität: **27.04.82 DE 3215591**

(43) Veröffentlichungstag der Anmeldung: **02.11.83 Patentblatt 83/44**

(45) Bekanntmachung des Hinweises auf die Patenterteilung: **24.07.85 Patentblatt 85/30**

(84) Benannte Vertragsstaaten: **BE DE FR GB IT NL**

(56) Entgegenhaltungen:
**EP - A - 0 054 817**
**DE - B - 2 530 001**
**US - A - 3 919 279**

(73) Patentinhaber: **BAYER AG, Konzernverwaltung RP Patentabteilung, D-5090 Leverkusen 1 Bayerwerk (DE)**

(72) Erfinder: **Engbert, Theodor, Dr., Goethestrasse 57c, D-4047 Dormagen (DE)**
Erfinder: **Hammen, Günter, Dr., Goethestrasse 67, D-4047 Dormagen (DE)**
Erfinder: **Knöfel, Hartmut, Dr., Dülmener Weg 21, D-5068 Odenthal-Erberich (DE)**
Erfinder: **König, Klaus, Dr., Heymannstrasse 50, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft ein verbessertes Verfahren zur kontinuierlichen thermischen Spaltung von Carbamidsäureestern, bei dem der zu spaltende Carbamidsäureester in flüssiger Form einen beheizten Rohrreaktor durchläuft und bei dem auf den Einsatz von verdünnend wirkenden Lösungsmitteln verzichtet werden kann.

Die thermische Spaltung von Carbamidsäureestern ist bereits seit langem bekannt. Wie z. B. Arbeiten von A.W. Hoffmann (Berichte der Deutschen Chemischen Gesellschaft, Jahrgang 1870, S. 653 ff.) und M. Métayer («Bull. Soc. Chim. France», Jahrgang 1951, S. 802 ff.) zeigen, sind diese Spaltungen reversibel, d. h.: Beim Abkühlen der heissen Reaktionsgemische rekombinieren die Isocyanate mit den Alkoholen und es werden erneut Carbamidsäureester gebildet. Daher bedarf es besonderer Massnahmen, um bei der thermischen Spaltung von Carbamidsäureestern die gebildeten Isocyanate und Alkohole getrennt gewinnen zu können.

In der US-PS Nr. 2409712 ist ein Verfahren beschrieben, das in mässiger Ausbeute die diskontinuierliche Herstellung monofunktioneller Isocyanate durch chargenweises Erhitzen der entsprechenden Carbamidsäureester auf Temperaturen von 150 bis 450° C gestattet. Zur Trennung der Spaltprodukte wird deren rasche Destillation oder das Einleiten in ein das Isocyanat und den Alkohol selektiv lösendes Lösungsmittelsystem, z. B. ein Cyclohexan/Wasser-Gemisch, vorgeschlagen. Dieses Verfahren ist zwar zur diskontinuierlichen Herstellung von monofunktionellen Isocyanaten im Labormassstab recht gut geeignet; die Beschränkung auf den diskontinuierlichen Betrieb sowie die nur mässigen Ausbeuten stehen jedoch einer technischen Nutzung im Wege. Ausserdem werden keine Wege aufgezeigt, die geeignet sein könnten, die bei der thermischen Spaltung von Carbamidsäureestern in mehr oder minder grossem Masse erfahrungsgemäss auftretende Nebenprodukt-Bildung zu unterdrücken bzw. deren nachteilige Auswirkungen auf den technischen Ablauf des Spaltungsprozesses zu beseitigen oder zumindest zu mildern.

Untersuchungen von H. Schiff (Berichte der Deutschen Chemischen Gesellschaft, Jahrgang 1870, S. 649 ff.) sowie von E. Dyer und G.C. Wright («J. Amer. Chem. Soc.», Bd. 81, Jahrgang 1959, S. 2138 ff.) zeigen, dass sich Carbamidsäureester bei thermischer Belastung ganz oder teilweise zu unterschiedlichen Produkten irreversibel zersetzen können. Unter anderem können dabei substituierte Harnstoffe, Biurete, Carbodiimide, Isocyanurate, sekundäre Amine, Olefine und/oder Kohlendioxid gebildet werden.

Um die Bildung unerwünschter Nebenprodukte bei der thermischen Spaltung von Carbamidsäureestern zu unterdrücken, sind verschiedene Verfahren entwickelt worden. Eine naheliegende Möglichkeit besteht selbstverständlich darin, die thermische Belastung bei der Spaltung klein zu halten. Im allgemeinen muss dann allerdings die thermische Spaltung in Gegenwart von Katalysatoren durchgeführt werden, da sonst zu geringe Raum/Zeit-Ausbeuten resultieren.

So werden in den US-PS Nrn. 2713591, 2692275, 2727020 und 4294774 sowie in der Japanischen Patentanmeldung Nr. 54-88201 (1979) Verfahren zur Herstellung von Isocyanaten durch thermische Spaltung von Carbamidsäureestern in Gegenwart basischer Katalysatoren beschrieben. Es ist jedoch bekannt, dass insbesondere basische Katalysatoren auch zu vermehrten irreversiblen Zersetzungsreaktionen von Carbamidsäureestern und Isocyanaten führen (vgl. z. B.: «J. Appl. Polym. Sci.», Bd. 16, Jahrgang 1972, S. 1212). Dementsprechend ergeben Verfahren mit basischen Katalysatoren nur dann annehmbare Isocyanatausbeuten, wenn die eingesetzten Carbamidsäureester durch entsprechende Substituenten gegen Zersetzung geschützt sind.

Eine weitere Möglichkeit zur Unterdrückung von Nebenreaktionen bei der thermischen Spaltung von Carbamidsäureestern besteht in der Verdünnung der Carbamidsäureester und/oder der Spaltprodukte durch inerte Verdünnungsmittel. In der US-PS Nr. 3919279, der DE-OS Nr. 2635490 und den Japanischen Patentanmeldungen Nrn. 54-39002 (1979) und 54-88222 (1979) sind Verfahren beschrieben, bei denen die thermische Spaltung von Carbamidsäureestern in inerten Lösungsmitteln, ggf. in Gegenwart bestimmter Katalysatoren, durchgeführt wird.

In den Verfahren, die in den DE-AS Nrn.2421503 und 2526193 beschrieben sind, werden ausser inerten Lösungsmitteln auch Trägergase, ggf. in Form von verdampften niedrigsiedenden Lösungsmitteln, mitverwendet. Die Verwendung von Lösungsmitteln bei der thermischen Spaltung von Carbamidsäureestern ist jedoch mit erheblichen Schwierigkeiten verbunden. Die eingesetzten Lösungsmittel müssen unter den Bedingungen der Thermolyse stabil und besonders gegenüber Isocyanaten inert sein; sie müssen mit den zu spaltenden Carbamidsäureestern gut mischbar sein, und schliesslich muss ihr Dampfdruck bei den angewandten Temperaturen so niedrig sein, dass sie während der Thermolyse im wesentlichen in der flüssigen Phase verbleiben. Durch diese Anforderungen ist die Auswahl an möglichen Lösungsmitteln sehr begrenzt. Insbesondere bei der Spaltung von Carbamidsäureestern mit hohem Molekulargewicht ist es schwierig, brauchbare und wohlfeile Lösungsmittel zu finden. Ausserdem bedeutet der Einsatz von Lösungsmitteln grundsätzlich eine Minderung der Raum/Zeit-Ausbeuten an Isocyanaten.

Schliesslich ist es bei hochsiedenden Lösungsmitteln aufwendig, aus den flüssigen Reaktionsgemischen die reinen Komponenten (Restmengen an Isocyanat und Carbamidsäureestern sowie an Lösungsmitteln) vom Rückstand destillativ abzutrennen, wie z. B. die DE-AS Nr. 2530001 lehrt. In jedem Fall erfordert die Aufarbeitung und Lagerung von inerten Lösungsmitteln einen bedeutenden zusätzlichen Aufwand.

Angewiesen auf die Verwendung von verdünnend wirkenden Lösungsmitteln sind die genannten Verfahren zur thermischen Spaltung von Carbamidsäureestern vor allem in den Fällen, in denen zur Spaltung mehrfunktionelle Carbamidsäureester eingesetzt werden, da ansonsten die hier im Reaktionsgemisch sich zwangsläufig bis zu einem gewissen Grade anreichernden Isocyanato-Urethane in starkem Masse zu unerwünschten Nebenprodukten weiterreagieren würden.

Bei der thermischen Spaltung monofunktioneller Carbamidsäureester kann dagegen ohne Inkaufnahme gravierender Ausbeuteverluste auf den Einsatz von verdünnend wirkenden Lösungsmitteln verzichtet werden, wie z. B. das in der älteren, nicht vorveröffentlichten deutschen Patentanmeldung P Nr. 3047898.9 (europäische Patentanmeldung Nr. 0054817) beschriebene Verfahren zur thermischen Spaltung monofunktioneller Carbamidsäurealkylester zeigt.

Dieses Verfahren ist allerdings mit dem Nachteil relativ langer Verweilzeiten der zu spaltenden Carbamidsäurealkylester behaftet.

Spaltverfahren, in denen Carbamidsäureester bei hohen Temperaturen (400 bis 600° C bzw. 350 bis 550° C) in der Gasphase gespalten werden, sind schliesslich in den US-PS Nrn. 3734941 und 3870739 beschrieben. Wichtig ist bei diesen Verfahren, dass die Verweilzeit der Gase in der Hochtemperaturzone klein bleibt, da sonst trotz der Verdünnung durch die Gasphase eine starke Zersetzung der Carbamidsäureester und/oder der Spaltprodukte aufgrund der hohen thermischen Belastung stattfindet. Kurze Verweilzeiten können jedoch dazu führen, dass die Ausbeuten an Isocyanaten unerwünscht klein bleiben. Zudem erfordern diese Verfahren einen hohen technischen Aufwand, da Gase wegen ihrer geringen Wärmeleitfähigkeit nur schwierig in kurzer Zeit aufgeheizt bzw. abgekühlt werden können.

Die Aufgabe der vorliegenden Erfindung war es, ein technisch praktikables und kostengünstiges Verfahren zur thermischen Spaltung von Carbamidsäureestern zur Verfügung zu stellen, das die Herstellung von Mono- und Polyisocyanaten in hohen Ausbeuten gestattet, und das nicht mit den Nachteilen der bekannten Verfahren behaftet ist.

Diese Aufgabe konnte durch das nachstehend näher erläuterte erfindungsgemässe Verfahren gelöst werden.

Gegenstand der vorliegenden Erfindung ist ein Verfahren zur kontinuierlichen thermischen Spaltung von N-monosubstituierten Carbamidsäureestern, deren Isocyanat- und/oder Hydroxylkomponente bei einem Druck von 0,01 bar einen unterhalb von 250° C liegenden Siedepunkt aufweisen, bei einem Druck von 0,001-20 bar und einer Temperatur von 150 bis 450° C und anschliessender Auftrennung der Spaltprodukte in eine vorwiegend aus der Isocyanatkomponente der Carbamidsäureester bestehende Fraktion A und eine vorwiegend aus der Hydroxylkomponente der Carbamidsäureester bestehende Fraktion B, dadurch gekennzeichnet, das man

a) die thermische Spaltung in einem Rohrreaktor durchführt, wobei die zu spaltenden Carbamidsäureester in flüssiger Form an der Innenwand des Rohrreaktors herablaufen oder an ihr entlanggeführt werden, und

b) die unter den Reaktionsbedingugen gasförmig anfallenden Spaltprodukte am Kopf des Rohrreaktors und die unter den Reaktionsbedingungen ggf. als Flüssigkeit anfallenden Spaltprodukte am Fusse des Rohrreaktors entnimmt.

Ausgangsmaterialien für das erfindungsgemässe Verfahren sind beliebige N-monosubstituierte Carbamidsäureester, deren Isocyanat- und/oder Hydroxylkomponente bei einem Druck von 0,01 bar einen unterhalb von 250° C liegenden Siedepunkt aufweisen. Vorzugsweise kommen solche Carbamidsäureester zum Einsatz, die bei dem jeweils im Reaktor vorherrschenden Druck einen mindestens 10° C über dem Siedepunkt des höhersiedenden Spaltprodukts liegenden Siedepunkt aufweisen, und deren Isocyanat- und Hydroxylkomponente ihrerseits bei Normaldruck einen mindestens 50° C voneinander verschiedenen Siedepunkt aufweisen. Für das erfindungsgemässe Verfahren besonders gut geeignete Carbamidsäureester sind solche der allgemeinen Formeln

$$R^1(NHCO_2R^2)_x \text{ oder } (R^1NHCO_2)_yR^2$$

wobei

$R^1$ jeweils für einen ggf. inerte Substituenten aufweisenden und/oder olefinisch ungesättigten aliphatischen Kohlenwasserstoffrest mit insgesamt 1 bis 18 Kohlenstoffatomen, einen ggf. inerte Substituenten aufweisenden und/oder olefinisch ungesättigten cycloaliphatischen Kohlenwasserstoffrest mit insgesamt 3 bis 18 Kohlenstoffatomen, einen ggf. inerte Substituenten aufweisenden araliphatischen Kohlenwasserstoffrest mit insgesamt 7 bis 18 Kohlenstoffatomen oder einen ggf. inerte Substituenten aufweisenden aromatischen Kohlenwasserstoffrest mit insgesamt 6 bis 18 Kohlenstoffatomen steht, wobei es sich bei den Resten $R^1$, den Formeln entsprechend, um x- bzw. einwertige Reste handelt,

$R^2$ jeweils für einen ggf. inerte Substituenten aufweisenden und/oder olefinisch ungesättigten aliphatischen Kohlenwasserstoffrest mit insgesamt 1 bis 18 Kohlenstoffatomen, einen ggf. inerte Substituenten aufweisenden und/oder olefinisch ungesättigten cycloaliphatischen Kohlenwasserstoffrest mit insgesamt 4 bis 18 Kohlenstoffatomen, einen ggf. inerte Substituenten aufweisenden araliphtischen Kohlenwasserstoffrest mit insgesamt 7 bis 18 Kohlenstoffatomen, oder einen ggf. inerte Substituenten aufweisenden aromatischen Kohlenwasserstoffrest mit insgesamt 6 bis 12 Kohlenstoffatomen, steht, wobei es sich bei den Resten $R^2$, den allgemeinen Formeln entsprechend, um ein- bzw. y-wertige Reste handelt,

x und y für gleiche oder verschiedene, ganze oder (im statistischen Mittel) gebrochene Zahlen von 1 bis 3 stehen,

mit der Massgabe, dass solche Carbamidsäureester der genannten allgemeinen Formeln eingesetzt werden, die bei dem im Reaktor vorliegenden Druck einen mindestens 10° C über dem Siedepunkt des höhersiedenden Spaltproduktes liegenden Siedepunkt aufweisen, und deren Isocyanat- und Hydroxylkomponenten ihrerseits bei Normaldruck einen mindestens 50° C voneinander verschiedenen Siedepunkt aufweisen.

Typische Beispiele geeigneter N-monosubstituierter Carbamidsäureester sind:
N-Methylcarbamidsäuredecylester,
N-Methylcarbamidsäuredodecylester,
N-Methylcarbamidsäure-octadecylester,
N-Ethylcarbamidsäure-octylester,
N-Ethylcarbamidsäure-octadecylester,
N-Ethylcarbamidsäure-2-(2-butoxyethoxy)-ethylester,
N-Butylcarbamidsäure-octadecylester,
N-Methoxymethylcarbamidsäure-dodecylester,
N-(2-Methoxyethyl)carbamidsäure-dodecylester,
N-Hexylcarbamidsäure-methylester,
N-Octylcarbamidsäure-ethylester,
N-(2-Methyl-1-hexenyl)carbamidsäureethylester,
N-Octadecylcarbamidsäure-butylester,
N-Cyclohexylcarbamidsäure-octadecylester,
N-Cyclohexylcarbamidsäure-ethylester,
N-Benzylcarbamidsäure-ethylester,
N-Allylcarbamidsäure-(2-ethylhexyl)ester,
N-(2-Phenylethyl)carbamidsäure-ethylester,
N-Phenylcarbamidsäure-methylester,
N-Phenylcarbamidsäure-propylester,
N-(4-Chlorphenyl)carbamidsäure-ethylester,
N-(3,4-Dichlorphenyl)carbamidsäure-butylester,
N-(4-Cyclohexylphenyl)carbamidsäure-ethylester,
N-(3-Methylphenyl)carbamidsäureethylester,
N-(4-Methoxycarbonylphenyl)carbamidsäure-ethylester,
N-1-Naphtylcarbamidsäure-methylester,
N-Phenylcarbamidsäure-octadecylester,
N-Phenylcarbamidsäure-dodecylester,
1,6-Bis(N-ethylcarbamoyloxy)hexan,
1,8-Bis-(N-propylcarbamoyloxy)octan,
1,6-Bis-(N-methoxymethylcarbamoyloxy)hexan,
1,12-Bis-(N-butylcarbamoyloxy)dodecan,
1,18-Bis-(N-2-butoxyethylcarbamoyloxy)octadecan,
1,12-Bis-(N-phenylcarbamoyloxy)dodecan,
1,12-Bis-(N-benzylcarbamoyloxy)octadecan,
4,4'-Bis-(N-butylcarbamoyloxy)-(2,2-dicyclohexylpropan),
1,1,1-Tris-[(N-ethylcarbamoyloxy)methyl]-propan,
1,3,6-Tris-(N-phenylcarbamoyloxy)hexan,
1,2-Bis-(cyclohexoxycarbonylamino)ethan,
1,4-Bis-(ethoxycarbonylamino)butan,
1,6-Bis-(ethoxycarbonylamino)hexan,
1,8-Bis-(ethoxycarbonylamino)octan,
1-(n-Butoxycarbonylamino)-3,3,5-trimethyl-5-(n-butoxycarbonylaminomethyl)cyclohexan,
1,4-Bis-(ethoxycarbonylamino)cyclohexan,
4,4'-Bis-(ethoxycarbonylamino)dicyclohexylmethan,
1-Methyl-2,4-bis-(ethoxycarbonylamino)-benzol,
1-Methyl-2,6-bis-(ethoxycarbonylamino)-benzol,
1,5-Bis-(butoxycarbonylamino)naphthalin,
1,3-Bis-(ethoxycarbonylaminomethyl)benzol,
2,4'-Bis-(ethoxycarbonylamino)diphenylmethan,
4,4'-Bis-(ethoxycarbonylamino)diphenylmethan.

Für das erfindungsgemässe Verfahren geeignet sind auch Gemische der schon beispielhaft genannten 2,4'- bzw. 4,4'-Bis-(alkoxycarbonylamino)diphenylmethane mit entsprechenden höherkernigen Homologen, in denen mehr als 2 Alkoxycarbonylamino-substituierte Benzolringe über Methylenbrücke miteinander verbunden sind. Solche «Carbamatgemische der Diphenylmethanreihe» entstehen beispielsweise bei der säurekatalysierten Kondensation von einfach Alkoxycarbonylamino-substituierten Benzolen mit Formaldehyd.

Geeignete Ausgangsmaterialien sind ferner N-monosubstituierte Carbamidsäurearylester wie beispielsweise
N-Methylcarbamidsäurephenylester,
N-Methylcarbamidsäure-(3-methylphenyl)ester,
N-Ethylcarbamidsäure-(2,4-dimethylphenyl)-ester,
N-Butylcarbamidsäure-(nonylphenyl)ester,
N-Cyclohexylcarbamidsäure-(dodecylphenyl)-ester,
N-Octadecylcarbamidsäurephenylester,
1,4-Bis-[(3-isopropyl-5-methylphenoxy)-carbonylamino]-butan,
4,4'-Bis-(phenoxycarbonylamino)diphenylmethan,
4,4'-Bis-(phenoxycarbonylamino)dicyclohexylmethan,
1,8-Bis-(phenoxycarbonylamino)-4-(phenoxycarbonylaminomethyl)octan,
1,3-Bis-(N-methylcarbamoyloxy)benzol,
4,4'-Bis-(N-ethylcarbamoyloxy)biphenyl oder
2,2'-Bis-(4-N-propylcarbamoyloxyphenyl)-propan.

Der Einsatz derartiger O-Aryl-carbamidsäureester ist allerdings gegenüber dem Einsatz der weiter oben beispielhaft genannten Carbamidsäureester mit aliphatischen, cycloaliphatischen oder araliphatischen Resten am Sauerstoffatom der Carbamidsäureester-Gruppe weniger bevorzugt.

Auch heterocyclische Struktureinheiten aufweisende Carbamidsäureester wie z. B. Tris-[6-(ethoxycarbonylamino)hexyl]-isocyanurat oder Tris-[6-(phenoxycarbonylamino)hexyl]-isocyanurat können beim erfindungsgemässen Verfahren als Ausgangsmaterialien eingesetzt werden.

Die für das erfindungsgemässe Verfahren geeigneten Carbamidsäureester sind im übrigen nach bekannten Methoden des Standes der Technik zugänglich. Ihre Herstellung erfolgt beispielsweise durch Umsetzung der entsprechenden Amine mit Chlorameisensäureestern, durch Carbonylierung entsprechender Nitroverbindungen in Gegenwart von Alkoholen, durch die bereits erwähnte Kondensationsreaktion von einfachen Carbamidsäureestern mit Formaldehyd oder mit anderen Aldehyden oder auch Ketonen, oder durch Umsetzung von Aminen mit Harnstoff und Alkoholen. Selbstverständlich kommt es bezüglich der Eignung der Carbamidsäureester für das erfindungsgemässe Verfahren nicht auf die Art ihrer Herstellung an.
entsprechender Nitroverbindungen in Gegenwart von Alkoholen, durch die bereits erwähnte kondensationsreaktion von einfachen Carbamidsäureestern mit Formaldehyd oder mit anderen Aldehyden oder auch Ketonen, oder durch Umsetzung von Aminen mit Harnstoff und Alkoholen. Selbstverständlich kommt es bezüglich der Eignung der Carbamidsäureester für das erfindungsgemässe Verfahren nicht auf die Art ihrer Herstellung an.

Es bestand nun die wesentliche, dem erfindungsgemässen Verfahren zugrundeliegende Beobachtung darin, dass sich mono- und polyfunktionelle Carbamidsäureester auch in unverdünnter und flüssiger Form glatt zu Isocyanaten und Hydroxyverbindungen spalten lassen, wenn die betreffenden Carbamidsäureester in einer dünnen Schicht an der Innenwand eines ausreichend hoch erhitzten Rohres entlang geführt werden. Charakteristisch für das erfindungsgemässe Verfahren und ausschlaggebend für eine erfolgreiche Unterdrückung von Nebenreaktionen ist dabei, dass die Verweilzeit der Carbamidsäureester in der heissen Zone des Spaltreaktors sehr kurz gehalten wird und die Spaltprodukte Isocyanat und Hydroxyverbindung rasch aus dem Reaktionsraum entfernt werden.

Darüber hinaus ist während der Spaltung auf die Einhaltung einer von der Art des zu spaltenden Carbamidsäureesters und evtl. verwendeter Hilfsstoffe abhängigen Mindesttemperatur zu achten, unterhalb derer eine vollständige Spaltung des eingespeisten Carbamidsäureesters nicht mehr gewährleistet ist.

Die Entfernung der Spaltprodukte aus dem Spaltreaktor kann, wie nachstehend noch detailliert erläutert werden wird, auf verschiedene Arten vorgenommen werden. Gemäss der bevorzugten Ausführungsform des erfindungsgemässen Verfahrens, bei der die Spaltprodukte Isocyanat und Hydroxyverbindung unter den Reaktionsbedingungen gasförmig anfallen, wird das gasförmige Produktgemisch am Kopf des Reaktors entnommen und, ggf. nach vorhergehender Abtrennung von mitgerissenem, nicht oder nur teilweise gespaltenem Ausgangsmaterial durch dessen Kondensation, an zwei hintereinander angeordneten, geeignet temperierten Dephlegmatoren selektiv kondensiert, so dass die hauptsächlich aus Isocyanat bestehende Fraktion A und die hauptsächlich aus der Hydroxyverbindung bestehende Fraktion B anfallen.

Gemäss einer weiteren Ausführungsform des erfindungsgemässen Verfahrens werden entweder die Hydroxyverbindung als gasförmiges und das Isocyanat als flüssiges Produkt oder die Hydroxyverbindung als flüssiges und das Isocyanat als gasförmiges Produkt dem Spaltreaktor entzogen. Auf die Auftrennung des den Spaltreaktor verlassenden Gasstromes kann in diesem Falle natürlich verzichtet werden, jedoch ist oftmals, insbesondere bei der nachstehend erwähnten Mitverwendung von hochsiedenden Flüssigkeiten oder sonstigen nicht flüchtigen Hilfs- und Zusatzmitteln, eine destillative Aufarbeitung der am Fuss des Reaktors entnommenen flüssigen Phase zwecks Reindarstellung des als Flüssigkeit anfallenden Spaltprodukts empfehlenswert.

Die für das erfindungsgemässe Verfahren geeigneten Spaltreaktoren können sehr unterschiedlich konstruiert sein; sie müssen lediglich so betrieben werden können, dass der in den Spaltraum eingespeiste Carbamidsäureester sich in einer dünnen Schicht auf der beheizten Rohrinnenwand verteilen kann und die gasförmigen und/oder flüssigen Spaltprodukte wie beschrieben aus dem Reaktionsraum entnommen werden können.

Die Verteilung des eingespeisten Carbamidsäureesters auf der Rohrinnenwand kann in senkrecht stehenden Rohrreaktoren ohne das Hinzuziehen besonderer Vorrichtungen geschehen, sofern der zu spaltende Carbamidsäureester über eine geeignete Vorrichtung, z. B. eine Düse, gleichmässig auf die Rohrwandung aufgetragen wird; sie kann aber auch unter Zuhilfenahme eines mechanischen Rührwerkes oder ähnlicher Einrichtungen bewerkstelligt werden. In nicht senkrecht stehenden Rohrreaktoren ist die Verwendung eines Rührwerkes oder einer anderen geeigneten Vorrichtung in den meisten Fällen unabdingbar.

Die zur Erzeugung bzw. Verbesserung des Flüssigkeitsfilmes eingesetzten Rührwerke können vorteilhaft auch zum Transport des an der Rohrwandung befindlichen Materials benutzt werden, sei es, dass sie die abwärts gerichtete Fliessbewegung des Flüssigkeitsfilmes hemmen, sei es, dass sie in schräg oder waagerecht liegenden Reaktoren den eingespeisten Carbamidsäureester bzw. die nicht gasförmigen Spalt- oder auch Zersetzungsprodukte über die Spaltzone hinweg zum Rohrende befördern.

Geeignete Spaltreaktoren sind z. B. als Fallfilmverdampfer arbeitende Glas-, Quarz- oder Metallrohre, mit schneckenartigen Rührwerken ausgestattete, ggf. zum Ende sich verjüngende Rohrreaktoren oder auch herkömmliche Dünnschichtverdampfer in diversen Ausführungsformen. Von diesen haben sich mit mechanischen Rührwerken ausgestattete Dünnschichtverdampfer als besonders günstig erwiesen.

Sofern bei Durchführung des erfindungsgemässen Verfahrens ein Dünnschicht- oder Fallfilmverdampfer zum Einsatz kommt, wird der zu spaltende Carbamidsäureester in flüssiger und vorzugsweise unverdünnter Form oberhalb der eigentlichen Spaltzone so in den Spaltreaktor eingebracht, dass er entweder ohne weitere Massnahmen oder nach Verteilung durch eine im Reaktor angebrachte Rührvorrichtung als dünner Flüssigkeitsfilm an der auf die optimale Spalttemperatur erhitzte Innenwand des Reaktors herabläuft.

Die Spalttemperatur ist so zu wählen, dass innerhalb der mittleren Verweildauer in der Spaltzone eine vollständige Spaltung des eingebrachten

Carbamidsäureesters eintritt. Bei einer mittleren Verweildauer von 0,01 bis 10 min, vorzugsweise 0,1 bis 5 min, hat sich für die meisten Fälle eine im Bereich von 150 bis 450° C, bevorzugt 200 bis 400° C, liegende Temperatur als ausreichend erwiesen.

Es kann u. Ü. auch von Vorteil sein, die Spaltzone in zwei oder mehrere unterschiedlich temperierte Abschnitte aufzuteilen.

Das erfindungsgemässe Spaltverfahren verläuft dann ordnungsgemäss, wenn im herablaufenden Flüssigkeitsfilm eine kontinuierliche Verarmung an eingespeistem Carbamidsäureester erfolgt und am Ende der Spaltzone kein oder nur sehr wenig Ausgangsmaterial ankommt. Das erfindungsgemässe Verfahren zur thermischen Spaltung von Carbamidsäureestern kann natürlich auch derart durchgeführt werden, dass man den eingebrachten Carbamidsäureester nur unvollständig spaltet. In diesem Falle erhält man am Fusse des Spaltreaktors ein Produkt, das überwiegend oder zumindest teilweise das unveränderte Ausgangsmaterial und/oder das zu Isocyanato- bzw. Hydroxyurethanen teilgespaltene Ausgangsmaterial enthält.

Diese Arbeitsweise kann dann interessant sein, wenn das so hergestellte, am Fusse des Reaktors entnommene Produkt für bestimmte Zwecke gebraucht werden kann, oder wenn mit dem nicht oder nur teilweise gespaltenen Ausgangsmaterial – wie später noch ausführlicher dargelegt wird – unerwünschte Nebenprodukte aus dem Spaltreaktor ausgeschleust werden können.

Im allgemeinen wird man jedoch eine vollständige Spaltung zu Isocyanat und Hydroxyverbindung anstreben.

Die bei der thermischen Spaltung entstehenden Produkte Isocyanat und Hydroxyverbindung können, wie bereits dargelegt, auf verschiedene Arten aus dem Spaltreaktor entfernt werden.

Sind beide Produkte unter den Spaltungsbedingungen gasförmig, so können diese nach Verlassen des Spaltreaktors und nach ggf. vorgenommener Abtrennung nicht umgesetzten Ausgangsmaterials durch selektive Kondensation in eine hauptsächlich das Isocyanat (Fraktion A) und eine hauptsächlich die Hydroxyverbindung enthaltende Fraktion B aufgetrennt werden. Als besonders gut geeignet zur raschen und effektiven Abtrennung bzw. selektiven Kondensation der Spaltprodukte hat sich eine aus 2 Dephlegmatoren bestehende Vorrichtung erwiesen, deren zuunterst angeordneter Dephlegmator so temperiert ist, dass an ihm die Bestandteile des gasförmigen Produktgemisches, die höher als die Spaltprodukte Isocyanat und Hydroxyverbindung sieden – also z. B. nicht oder nur teilweise umgesetztes Ausgangsmaterial – weitestgehend kondensiert werden und so in den Spaltreaktor zurücklaufen können, die Spaltprodukte Isocyanat und Hydroxyverbindung aber durchgelassen werden.

Am 2. Dephlegmator wird das am Kopf des 1. Dephlegmators entweichende Gasgemisch dagegen so teilkondensiert, dass als Kondensat ein hauptsächlich das höhersiedende der beiden Spaltprodukte enthaltendes Produkt entsteht und der den 2. Dephlegmator passierende Teil des Gasgemisches hauptsächlich aus der tiefersiedenden Komponente der beiden Spaltprodukte besteht.

Das am 2. Dephlegmator gebildete Kondensat, das je nach Art des eingesetzten Carbamidsäureesters entweder vorwiegend aus dem Isocyanat $R^1(NCO)_x$ oder aus der Hydroxyverbindung $R^2(OH)_y$ besteht, wird in einer Vorlage aufgefangen und kann zur weiteren Reinigung nochmals destilliert werden. In gleicher Weise kann das am Kopf des 2. Dephlegmators entweichende, hauptsächlich das niedriger siedende Spaltprodukt enthaltende gasförmige Produktgemisch, nach Verflüssigung an einem entsprechend temperierten Kondensator in einem Zwischenbehälter gesammelt und nochmals destilliert werden.

Die bei der destillativen Reindarstellung der Isocyanate $R^1(NCO)_x$ und Hydroxyverbindungen $R^2(OH)_y$ entstehenden, grossteils aus nicht oder nur teilweise umgesetztem Ausgangsmaterial bestehenden Destillationssümpfe können in den Spaltreaktor zurückgeführt und dort erneut dem Prozess der thermischen Spaltung unterworfen werden.

Für diese Variante des erfindungsgemässen Verfahrens geeignete Carbamidsäureester sind insbesondere solche der o. g. allgemeinen Formeln, deren Isocyanat- und Hydroxylkomponenten unter den Reaktionsbedingungen gasförmig sind und sich in ihren Siedepunkten, wie oben ausgeführt, deutlich voneinander und vom Siedepunkt des Carbamidsäureesters unterscheiden.

Weisen der zu spaltende Carbamidsäureester und eines der bei der thermischen Spaltung entstehenden Spaltprodukte nur geringe Siedepunktsdifferenzen auf, wie z. B. 10 bis 15° C, ein Fall, der z. B. bei Carbamidsäureestern auf Basis niedrig siedender Monoisocyanate auftreten kann, so empfiehlt sich eine Verfahrensweise, bei der die Abtrennung des nicht umgesetzten Carbamidsäureesters vom ähnlich hochsiedenden Spaltprodukt bei einer in der Nähe oder am Siedepunkt des hochsiedenden Spaltproduktes liegenden Temperatur unter teilweisem Rückfluss des hochsiedenden Spaltproduktes erfolgt. Diese Trennoperation kann z. B. mit gutem Erfolg in einer zwischen dem Spaltreaktor und dem 1. Dephlegmator angeordneten, beheizten Kolonne erfolgen.

Solche Carbamidsäureester sowie Carbamidsäureester, bei denen eines der beiden Spaltprodukte nicht oder nur schwer destillierbar ist, können jedoch auch nach einer anderen Verfahrensweise gespalten werden, bei der das hochsiedende, d. h. das schwer oder nicht destillierbare Spaltprodukt in flüssiger Form am Fuss des Reaktors entnommen wird, und das unter den Reaktionsbedingungen gasförmige Spaltprodukt wie oben beschrieben am Kopfe des Reaktors entnommen und anschliessend kondensiert wird. Bei dieser Verfahrensweise ist durch eine geeignete Wahl der Verfahrensparameter, vor allem durch die Wahl der Spalttemperatur, sicherzustellen, dass innerhalb der Verweilzeit, in der der Flüssigkeitsfilm die

Spaltzone durchläuft, die Spaltung des eingebrachten Carbamidsäureesters in der gewünschten Weise abläuft. Nur dann kann ein von nicht umgesetztem Ausgangsmaterial weitgehend freies Produkt am Fusse des Spaltreaktors entnommen werden.

Dieses Produkt, das – abgesehen von evtl. vorhandenen Nebenbestandteilen – je nach Art des eingesetzten Carbamidsäureesters entweder ein mono- bzw. polyfunktionelles Isocyanat oder eine mono- bzw. polyfunktionelle Hydroxyverbindung ist, kann bei ausreichender Reinheit entweder als solches der gewünschten Verwendung zugeführt werden oder erst noch geeigneten Reinigungsoperationen unterzogen werden.

Welche der genannten Verfahrensvarianten zur Entfernung der Spaltprodukte aus dem Spaltreaktor und deren Auftrennung angewandt werden, hängt von der Art des zu spaltenden Carbamidsäureesters, vom in der Spaltapparatur herrschenden Druck, und der Spalttemperatur ab.

Alle genannten Varianten des erfindungsgemässen Verfahrens können bei einem im Bereich von 0,001 bis 20 bar liegenden Druck durchgeführt werden; in der Praxis wird jedoch ein zwischen 0,01 und 1,3 bar liegender Druck bevorzugt angewendet. Innerhalb dieses Druckbereiches sind bei einer zwischen 150 und 450° C liegenden Temperatur fast alle in Frage kommenden Hydroxyverbindungen und der Grossteil der in Betracht kommenden Isocyanate destillierbar, so dass das erfindungsgemässe Verfahren in der Mehrzahl der Fälle nach der zuerst beschriebenen Verfahrensvariante, in der beide Spaltprodukte gasförmig dem Spaltreaktor entnommen werden, durchgeführt werden kann.

Ein wichtiger Sonderfall ist die thermische Spaltung von Di- und Polycarbamidsäureestern der Diphenylmethanreihe, die z. B. bei der säurekatalysierten Kondensation von N-Phenylcarbamidsäurealkylestern mit Formaldehyd entstehen. Da bei der thermischen Spaltung dieser Carbamidsäureester nicht destillierbare Polyisocyanate entstehen, kann hier das erfindungsgemässe Verfahren nur nach der 2. Variante durchgeführt werden, bei der das höhersiedende Spaltprodukt am Fusse des Reaktors entnommen wird.

Sofern bei der Durchführung des erfindungsgemässen Verfahrens schwerflüchtige Nebenprodukte, die unter den Reaktionsbedingungen nicht destillierbar sind, gebildet werden, können diese auf verschiedene Weise aus dem Spaltreaktor entfernt werden.

Bei Durchführung der thermischen Spaltung nach der 2. Verfahrensvariante, bei der eines der beiden Spaltprodukte am unteren Ende des Spaltreaktors entnommen wird, werden entwaige schwerflüchtige Nebenprodukte mit dem nach unten ablaufenden Spaltprodukt entfernt, so dass ernsthafte Probleme bei der Nebenprodukt-Ausschleusung allenfalls bei der Bildung sehr schwerlöslicher Nebenprodukte zu erwarten sind.

Die Abtrennung der Nebenprodukte aus den vorwiegend das Isocyanat bzw. vorwiegend die Hydroxyverbindung enthaltenden, am Fusse des Spalreaktors anfallenden Rohprodukte kann bei destillierbaren Spaltprodukten im Zuge der destillativen Reindarstellung des Isocyanats bzw. der Hydroxyverbindung erfolgen. Im Falle nicht destillierbarer Spaltprodukte ist eine Reinigung – sofern überhaupt notwendig – nach anderen Methoden, z. B. durch Filtration oder Extraktion, möglich.

Einer besonderen Aufmerksamkeit bedarf das Problem der Nebenprodukt-Ausschleusung vor allem dann, wenn die thermische Spaltung nach der 1. Verfahrensvariante, in der praktisch die Gesamtmenge des eingespeisten Materials den Spaltreaktor gasförmig verlässt, durchgeführt wird. Unter diesen Bedingungen können sich vor allem feste oder sehr zähflüssige Nebenprodukte im unteren Teil des Spaltreaktors ablagern und dadurch zu schwerwiegenden Störungen des Spaltbetriebes führen.

Eine Möglichkeit zur Verhinderung oder Verminderung solcher Ablagerungen besteht z. B. darin, dass man durch eine Temperaturanhebung im unteren Teil des Spaltreaktors eine Erweichung bzw. Verflüssigung und damit einen besseren Ablauf der gebildeten Nebenprodukte erreicht.

In vielen Fällen reicht eine Temperaturerhöhung allein nicht aus. Als weitere geeignete Massnahme ist daher die Mitverwendung einer unter den Spaltbedingungen schwerflüchtigen und möglichst inerten Flüssigkeit, d. h. Lösungsmittels oder Weichmachers zu nennen, die entweder zusammen mit dem zu spaltenden Carbamidsäureester oder separat über eine eigene Dosiervorrichtung an geeigneter Stelle in den Spaltreaktor eingeschleust wird. Die zur Ausschleusung der gebildeten schwerflüchtigen Nebenprodukte einzusetzende Menge eines bestimmten Lösungsmittels oder Weichmachers hängt in starkem Masse von der Struktur des zu spaltenden Carbamidsäureesters sowie von den darin vorhandenen Nebenbestandteilen ab. Im allgemeinen kommen, falls überhaupt, Mengen von 0,1 bis 50 Gew.-%, bevorzugt 1 bis 10 Gew.-%, bezogen auf die Menge des eingesetzten Carbamidsäureesters, zur Anwendung.

Nach der Entnahme am Fusse des Reaktors können die im Lösungsmittel bzw. Weichmacher in gelöster und/oder auch ungelöster Form vorliegenden Nebenprodukte, z. B. durch Filtration, Destillation oder Extraktion, abgetrennt und die gereinigten Lösungsmittel bzw. Weichmacher erneut zur Nebenprodukt-Ausschleusung eingesetzt werden.

Geeignete Lösungsmittel bzw. Weichmacher sind beispielsweise:

Aliphatische und cycloaliphatische Kohlenwasserstoffe, wie z. B. Dodecan, Octadecan, Decalin, höhere Alkene und überwiegend Alkane enthaltende hochsiedende Erdölfraktionen; aromatische Kohlenwasserstoffe wie z. B. Dodecylbenzol, Methylnaphthalin, Benzylnaphthalin, Terphenyl, Diphenylmethan oder Biphenyl, chlorierte oder Ethergruppen enthaltende aromatische Verbindungen wie z. B. Dichlorbenzol, Chlornaphthalin, Dichlorbenzylnaphthalin, Benzylchlornaphthalin, Diphenylether, Methylnaphthylether oder

Pentylnaphthylether; Sulfone wie Diphenylsulfon oder Naphthylphenylsulfon, ferner Ester organischer und anorganischer Säuren wie z. B. Dibutylphthalat, Dioctylphthalat, Phenylbenzoat, Dioctyladipat, Dioctylsebacat, Triphenylphosphat oder Trikresylphosphat.

In manchen Fällen kann es auch von Vorteil sein, solche Verbindungen zur Nebenprodukt-Ausschleusung einzusetzen, die zwar unter Normalbedingungen mit Isocyanaten reagieren, unter den Reaktionsbedingungen aber weitgehend frei vorliegen.

Solche Verbindungen sind z. B.:

Hochsiedende Alkohole und Phenole wie Octadecylalkohol, Benzylalkohol, 1,6-Hexandiol, Tetraethylenglykol, Polyethylen- und Polypropylenglykole, Phenol, Kresol, Nonylphenol oder Resorcinol sowie andere üblicherweise als Blockierungsmittel für Isocyanate verwendete Verbindungen.

Eine weitere Möglichkeit zur Ausschleussung von unerwünschten, schwerflüchtigen Nebenprodukten besteht darin, das bei der thermischen Spaltung entstehende höhersiedende Spaltprodukt nicht vollständig zu verdampfen und als Gas aus dem Spaltreaktor zu entfernen, sondern einen gewissen Teil desselben in flüssigen Form an der Wandung des Spaltreaktors herablaufen zu lassen und damit die ggf. entstandenen schwerflüchtigen Nebenprodukte aus dem Reaktionsraum auszuschleusen.

Ferner besteht die Möglichkeit, die Reaktionsbedingungen so einzustellen, dass ein mehr oder weniger kleiner Teil des eingespeisten Carbamidsäureesters nicht oder nicht vollständig gespalten wird und damit als Lösungsmittel für die ggf. entstandenden Nebenprodukte fungieren kann.

Sofern es erwünscht ist, können aus dem am Fusse des Spaltreaktors anfallenden Stoffgemisch der zur Nebenprodukt-Ausschleusung verwendete Teil des Ausgangsmaterials bzw. Spaltproduktes durch eine geeignete Trennoperation, z. B. durch Filtration, Extraktion oder Destillation zurückgewonnen werden.

Um die beim Spaltprozess entstehenden Produkte möglichst rasch und effektiv aus dem Reaktionsraum zu entfernen und um die Spaltprodukte an der Rekombination zu hindern, kann es zweckmässig sein, in den Spaltreaktor ein inertes Gas oder eine bei Normalbedingungen flüssige, unter den Reaktionsbedingungen aber gasförmige Verbindung, deren Trennung von den Spaltprodukten keine Probleme bereitet, einzuleiten. Sofern beide Spaltprodukte als Gase aus dem Spaltreaktor entnommen werden, kann es von Vorteil sein, die Einleitung des inerten Gases bzw. der niedrigsiedenden inerten Flüssigkeit an einer Stelle oberhalb der Spaltzone, aber unterhalb des Dephlegmatoren-Systems vorzunehmen. Dadurch wird eine unnötige Abkühlung des Reaktionsraumes vermieden, die Rekombination der Spaltprodukte im Dephlegmatoren-System jedoch nach wie vor wirksam behindert.

Das erfindungsgemässe Verfahren kann ferner unter zusätzlicher Verwendung von an sich bekannten Spaltungskatalysatoren, wie sie beispielsweise in der DE-OS Nr. 2635490 oder der US-PS Nr. 3919279 beschrieben sind, durchgeführt werden. Diese Katalysatoren werden, falls überhaupt, in Mengen von 0,001 bis 5 Gew.-%, vorzugsweise 0,01 bis 1 Gew.-%, bezogen auf das Gewicht des eingesetzten Carbamidsäureesters eingesetzt.

Vorzugsweise werden solche Katalysatoren des Standes der Technik eingesetzt, deren Siedepunkt deutlich oberhalb der Siedepunkte der gasförmig aus dem Spaltreaktor zu entfernenden Spaltprodukte liegen, die aber noch ggf. zusammen mit den zur Nebenprodukt-Ausschleusung verwendeten Lösungsmitteln oder Weichmachern destilliert und so für einen erneuten Einsatz zurückgewonnen werden können. Im Falle der Herstellung von nicht destillierbaren Isocyanaten empfiehlt sich, sofern überhaupt Katalysatoren eingesetzt werden, die Verwendung solcher Katalysatoren, deren vollständige destillative Abtrennung vom im Destillationssumpf verbleibenden Isocyanat leicht zu bewerkstelligen ist.

Als zusätzliche Hilfsstoffe können schliesslich auch stabilisierend wirkende Verbindungen bei Durchführung des erfindungsgemässen Verfahrens eingesetzt werden. Diese stabilisierend wirkenden Hilfsstoffe aus der Gruppe der Säurechloride und der alkylierend wirkenden Verbindungen wie beispielsweise Isophthalsäuredichlorid oder Toluolsulfonsäuremethylester werden, falls überhaupt, in ähnlicher Weise wie die Katalysatoren eingesetzt, und kommen wie diese im allgemeinen in Mengen von 0,001 bis 5 Gew.-%, vorzugsweise 0,01 bis 1 Gew.-%, bezogen auf das Gewicht des eingespeisten Carbamidsäureesters zu Anwendung.

In Anbetracht des oben zitierten Standes der Technik muss es als äusserst überraschend bezeichnet werden, dass das erfindungsgemässe Verfahren die thermische Spaltung von Carbamidsäureestern unter Gewinnung hoher Ausbeuten an den Carbamidsäureestern zugrundeliegenden Isocyanaten gestattet. Besonders hervorzuheben ist hierbei, dass das erfindungsgemässe Verfahren auf eine Vielzahl sehr unterschiedlich aufgebauter Carbamidsäureester angewendet werden kann. So sind nicht nur monofunktionelle Carbamidsäureester mit sehr kurzer Verweilzeit spaltbar, sondern es können auch Carbamidsäureester aus mehrfunktionellen Isocyanaten und monofunktionellen Hydroxyverbindungen bzw. aus höherwertigen Hydroxyverbindungen und monofunktionellen Isocyanaten in Abwesenheit von Lösungsmitteln mit gutem Erfolg zu den entsprechenden Isocyanaten und Hydroxyverbindungen gespalten werden. Dies ist insbesondere deswegen überraschend, weil nach den bisher beschriebenen Verfahren zur Spaltung mehrfunktioneller Carbamidsäureester (vgl. z. B. DE-OS Nr. 2635490, DE-AS Nrn. 2421503 und 2526193, sowie US-PS Nr. 3919279) nur dann hohe Ausbeuten an Isocyanaten erhältlich sind, wenn verdünnend wirkende Lösungsmittel in hohem Überschuss eingesetzt werden. Die Möglichkeit, bei der Durchführung des erfindungsgemässen Verfahrens auf den Einsatz von Lösungsmitteln ganz oder teilweise zu verzichten, bedeutet daher einen erheblichen Vorteil gegenüber den bisher bekannten Verfahren des Standes der Technik.

Die folgenden Beispiele dienen der weiteren Erläuterung des erfindungsgemässen Verfahrens. Alle Prozentangaben beziehen sich, soweit keine anderslautende Mitteilung gemacht wird, auf Gewichtsprozente.

*Beispiel 1:*

*Thermische Spaltung von N-Methylcarbamidsäureoctadecylester*

Die zur Spaltung benutzte Apparatur war wie folgt konstruiert:

Der eigentliche Spaltteil bestand aus einem rohrförmigen Dünnschichtverdampfer mit einer 350 $cm^2$ grossen Verdampferfläche, in dem sich ein schnellaufender, mit beweglichen, bis an die Wandung des Dünnschichtverdampfers reichenden Metallblättern ausgestatteter Rührer befand.

Zur Einspeisung des Carbamidsäureesters war am Kopf des Dünnschichtverdampfers ein beheizbarer Tropftrichter angebracht; für die Produktentnahme war je ein Auslass am Fuss und Kopf des Dünnschichtverdampfers vorhanden. Der untere Auslass war über einen Absperrhahn an einen Auffangkolben angeschlossen und der oberen über ein wärmeisoliertes Rohr mit einer beheizbaren Vigreux-Kolonne verbunden. Auf diese Kolonne waren zwei hintereinander angeordnete Schlangenkühler mit je einem Entnahmeboden zur Abführung der in den Kühlern gebildeten Kondensate aufgesetzt.

Über eine mit Aceton/Trockeneis gekühlte Falle war die Spaltapparatur an die Aussenluft angeschlossen.

Während der Spaltung wurde der geschmolzene N-Methylcarbamidsäureoctadecylester über den auf 100° C thermostatisierten Tropftrichter mit einer Rate von 50 g/h in den mit 360° C heissem Wärmeträgeröl erhitzten Dünnschichtverdampfer kontinuierlich eingespeist, wobei man die elektrisch beheizbare Vigreux-Kolonne auf eine Temperatur von 360 bis 370° C, den unteren Schlangenkühler auf eine Temperatur von 60° C sowie den oberen Kühler auf eine Temperatur von −20° C thermostatisierte.

Der Druck in der Apparatur betrug während der Spaltung 1013 mbar.

Der Absperrhahn am unteren Auslass des Dünnschichtverdampfers war während des Spaltbetriebes geschlossen und wurde nur zur Entnahme der sich während der Spaltung am Fuss des Dünnschichtverdampfers ansammelnden Stoffe zeitweilig kurz geöffnet.

Während der Spaltung wurde ferner ein Strom getrockneten Stickstoffs kurz unterhalb des unteren Entnahmebodens mit einer Rate von etwa 10 ml/min in die Apparatur eingeleitet und nach Durchlaufen des Dephlegmatoren-Systems und der mit Aceton/Trockeneis gekühlten Falle ins Freie abgeführt.

Der am zweiten Entnahmeboden angeschlossene Auffangkolben für das überwiegend Methylisocyanat enthaltende Kondensat war auf −20° C gekühlt, während der Kolben zur Aufnahme des am ersten Kühler gebildeten Kondensates sowie der Kolben am Fuss des Dünnschichtverdampfers Raumtemperatur aufwiesen.

Unter diesen Bedingungen wurde der in den Dünnschichtverdampfer eingespeiste und vom Rührer gleichmässig auf die Innenwandung verteilte N-Methylcarbamidsäureoctadecylester während des Herablaufens praktisch vollständig zu Methylisocyanat und Octadecanol gespalten.

Am Fusse des Dünnschichtverdampfers sammelte sich im Laufe der Spaltung ein Produktgemisch an, das neben geringen Megen an nicht umgesetztem Ausgangsmaterial und nicht näher charakterisierten, bei der Spaltung entstandenen Nebenprodukten überwiegend aus Octadecanol bestand. Dieses Material wurde während der Spaltung, zum überwiegenden Teil aber nach Beendigung der Spaltung, diskontinuierlich abgelassen.

Der bei der Spaltung entstehende gasförmige Produktstrom, der über den oberen Auslass in die beheizte Vigreux-Kolonne geführt wurde, kondensierte dort teilweise, so dass ein vorwiegend aus Octadecanol und N-Methylcarbamidsäureoctadecylester bestehendes Kondensat entstand, das dem aufsteigenden Gasstrom entgegen- und in den Dünnschichtverdampfer zurückfloss.

Das am Kopf der Vigreux-Kolonne entweichende Gasgemisch wurde an den beiden übereinander angeordneten Schlangenkühlern so aufgetrennt, dass das am ersten Kühler gebildete Kondensat vorwiegend aus Octadecanol und das am zweiten Kühler gebildete Kondensat vorwiegend aus Methylisocyanat bestand.

Innerhalb von 7 h wurden auf diese Weise 350 g an N-Methylcarbamidsäureoctadecylester gespalten, und dabei 57 g an rohem Methylisocyanat (Reinheit laut GC: 99%) sowie 277 g an rohem Octadecanol (Zusammensetzung laut GC: 92 Gew.-% Octadecanol, 8 Gew.-% N-Methylcarbamidsäureoctadecylester) erhalten. Ferner wurden 16 g eines bräunlichen Materials am Fuss des Dünnschichtverdampfers entnommen, das zu 12 Gew.-% aus N-Methylcarbamidsäureoctadecylester und im übrigen vorwiegend aus Octadecanol bestand.

Daraus errechnet sich hinsichtlich der Methylisocyanatbildung eine Ausbeute von 92,5% der Theorie und eine Selektivität von 99,3 % der Theorie.

*Beispiel 2:*

*Thermische Spaltung von 1-Methyl-2,4-bis-(ethoxycarbonylamino)benzol*

Es wurde die in Beispiel 1 beschriebene Apparatur benutzt; anstelle der Vigreux-Kolonne war jedoch ein Schlangenkühler eingebaut, der während der Spaltung der Abtrennung der Spaltprodukte vom nicht bzw. partiell gespaltenen Ausgangsmaterial diente und dazu mit Wärmeträgeröl auf eine Temperatur von 165° C gehalten wurde.

Der Druck in der Apparatur war auf 40 mbar eingestellt und die für die Kondensation der Spaltprodukte vorgesehenen Kühler wurden mit Leitungswasser bzw. mit gekühltem Methanol auf 20° C bzw. auf −20° C gekühlt. Ein Trägergas wurde nicht verwendet.

143,2 g 1-Methyl-2,4-bis-(ethoxycarbonylamino)benzol wurden vor Beginn der Spaltung aufgeschmolzen, im Tropftrichter bei einer Temperatur von 135° C vorgelegt und anschliessend innerhalb von 3 h kontinuierlich in den mit 300° C heissem Wärmeträgeröl erhitzten Dünnschichtverdampfer eingespeist. Parallel dazu wurden über einen zweiten Tropftrichter 28,0 g Trikresylphosphat kontinuierlich eindosiert.

Unter diesen Bedingungen wurde das eingespeiste 1-Methyl-2,4-bis-(ethoxycarbonylamino)-benzol in der Spaltzone des Dünnschichtverdampfers weitgehend zu Ethanol und 2,4-Toluylendiisocyanat gespalten. Durch partielle Spaltung des Ausgangsmaterials entstandene Isocyanato-Urethane wurden, soweit sie mit den anderen Spaltprodukten am oberen Auslass aus dem Dünnschichtverdampfer entwichen, überwiegend am ersten, auf 165° C thermostatisierten Kühler kondensiert und so in den Dünnschichtverdampfer zurückgeführt.

Das den ersten Kühler verlassende Gasgemisch wurde in den zwei darauffolgenden Kühlern so kondensiert, dass ein vorwiegend das 2,4-Toluylendiisocyanat und ein vorwiegend das Ethanol enthaltendes Kondensat entstanden.

Nach Beendigung der Spaltung wurden folgenden Daten ermittelt:

Es wurden 41,1 g an vorwiegend Ethanol enthaltendem Kondensat erhalten; dieses bestand zu 94,4 Gew.-% aus Ethanol und zu 5,5 Gew.-% aus 1-Methyl-2,4-bis-(ethoxycarbonylamino)-benzol. Das vorwiegend das Isocyanat enhaltende Kondensat wurde in einer Menge von 100,5 g erhalten und bestand zu 52,7 Gew.-% aus 2,4-Toluylendiisocyanat, zu 0,9 Gew.-% aus 1-Methyl-2,4-bis-(ethoxycarbonylamino)benzol, zu 44,4 Gew.-% aus 1-Methyl-2-(ethoxycarbonylamino)-4-isocyanato-benzol bzw. 1-Methyl-2-isocyanato-4-(ethoxycarbonylamino)benzol und zu 2,0 Gew.-% aus Trikresylphosphat.

Am Fuss des Dünnschichtverdampfers wurden insgesamt 27,5 g einer trüben, bräunlichen Flüssigkeit aufgefangen, von der 85 Gew.-% im Vakuum bei 1 mbar destilliert werden konnten.

Aus den genannten Daten errechnet sich hinsichtlich der Bildung des 2,4-Toluylendiisocyanats eine Ausbeute von 56,5% der Theorie und eine Selektivität von 96,4% der Theorie sowie hinsichtlich der Bildung des Ethanols eine Ausbeute von 78,3% der Theorie und eine Selektivität von 99,4% der Theorie.

*Beispiel 3:*

*Thermische Spaltung eines Gemisches aus 80% 1-Methyl-2,4-bis-(ethoxycarbonylamino)benzol und 20% 1-Methyl-2,6-bis-(ethoxycarbonylamino)benzol in Gegenwart eines Katalysators*

Es wurde die in Beispiel 2 beschriebene Spaltapparatur benutzt; der installierte Dünnschichtverdampfer wies jedoch eine vergrösserte Verdampferfläche von 900 $cm^2$ auf.

Die thermische Spaltung wurde bei einem Druck von 10 mbar durchgeführt; die Beheizung des Dünnschichtverdampfers erfolgte mit 240° C heissem Wärmeträgeröl und die Schlangenkühler des Dephlegmatoren-Systems waren auf 135° C bzw. auf 20° C bzw. auf −25° C thermostatisiert.

Während der 3stündigen Spaltung wurde mittels des auf 135° C erhitzten Tropftrichters eine homogene Mischung aus 645 g des o.g. Carbamidsäureesters, 32 g Phenyl-(1,2,3,4-tetrahydro-5-(6)-naphthyl)sulfon und 2,0 g Dibutylzinndilaurat kontinuierlich in den Dünnschichtverdampfer eindosiert.

Der gasförmige Produktstrom wurde wie in Beispiel 2 beschrieben in eine vorwiegend aus 2,4-Toluylendiisocyanat bzw. 2,6-Toluylendiisocyanat und eine vorwiegend aus Ethanol bestehende Fraktion aufgespalten, während die nicht gasförmigen Produktanteile zusammen mit dem eingesetzten Lösungsmittel am Fuss des Dünnschichtverdampfers ausgeschleust wurden.

Das nach Beendigung der Spaltung erhaltenen, überwiegend aus Ethanol bestehende Kondensat betrug 243,2 g und bestand zu 63,4 Gew.-% aus Ethanol und zu 36,6 Gew.-% aus 1-Methyl-2,4-(2,6)-bis-(ethoxycarbonylamino)benzol, während das isocyanathaltige Kondensat in einer Menge von 395,7 g erhalten wurde und zu 56,4 Gew.-% aus 2,4-(2,6-Toluylendiisocyanat, zu 39,1 Gew.-% aus 1-Methyl-2-(4)-(ethoxycarbonylamino)-4-(2)-isocyanatobenzol bzw. 1-Methyl-2-(ethoxycarbonylamino)-6-isocyanatobenzol, zu 3,1 Gew.-% aus 1-Methyl-2,4-(2,6-bis-(ethoxycarbonylamino)benzol und zu 5,5 Gew.-% aus Phenyl-(1,2,3,4-tetrahydro-5-(6)-naphthyl)sulfon bestand.

Die Menge des am Fuss des Dünnschichtverdampfers erhaltenen trüben und bräunlich gefärbten Produktes betrug 38 g.

Aus den genannten Daten errechnet sich hinsichtlich der Bildung des 2,4-(2,6)-Toluylendiisocyanats eine Ausbeute von 52,9% der Theorie und eine Selektivität von 97,6% der Theorie sowie hinsichtlich der Ethanolbildung eine Ausbeute von 69,1% der Theorie und eine Selektivität von 99,2% der Theorie.

*Beispiel 4:*

*Thermische Spaltung von 1-(Ethoxycarbonylamino)-3,3,5-trimethyl-5-(ethoxycarbonylaminomethyl)cyclohexan*

Zur Spaltung wurde die in Beispiel 2 beschriebene Apparatur eingesetzt; die Beheizung erfolgte mit 290° C heissem Wärmeträgeröl. Der Schlangenkühler am oberen Auslass des Dünnschichtverdampfers war auf 170° C erhitzt, der Druck in der Apparatur war auf 12 mbar eingestellt.

Während der Spaltung wurde durch den auf 120° C erhitzten Tropftrichter eine homogene Mischung aus 400 g 1-(Ethoxycarbonylamino)-3,3,5-trimethyl-5-(ethoxycarbonylaminomethyl)cyclohexan, 30 g Dichlorbenzylnaphthalin

und 3 g Dibutylzinndilaurat innerhalb von 5 h kontinuierlich eindosiert.

Die gasförmigen Spaltprodukte wurden wie in Beispiel 2 beschrieben in eine vorwiegend aus 1-Isocyanato-3,3,5-trimethyl-5-isocyanatomethylcyclohexan und eine vorwiegend aus Ethanol bestehende Fraktion aufgetrennt; die nicht gasförmigen Produkte und das eingesetzte Lösungsmittel wurden am Fusse des Dünnschichters ausgeschleust.

An Spaltprodukten wurden erhalten:
115,4 g Ethanolfraktion (77,1 Gew.-% Ethanol, 22,6 Gew.-% 1-(Ethoxycarbonylamino)-3,3,5-trimethyl-5-(ethoxycarbonylaminomethyl)cyclohexan,
0,3 Gew.-% Dichlorbenzylnaphthalin),
264,4 g Isocyanatfraktion (55,5 Gew.-% 1-Isocyanato-3,3,5-trimethyl-5-(isocyanatomethyl)-cyclohexan,
43,2 Gew.-% 1-(Ethoxycarbonylamino)-3,3,5-trimethyl-5-(isocyanatomethyl)cyclohexan bzw. 1-Isocyanato-3,3,5-trimethyl-5-(ethoxycarbonylaminomethyl)cyclohexan,
1,3 Gew.-% Dichlorbenzylnaphthalin).

Als Rückstand wurden 39,8 g am Fusse des Dünnschichtverdampfers ausgeschleust.

Bezüglich der Diisocyanatbildung berechnet sich daraus eine Ausbeute von 51,8% der Theorie und eine Selektivität von 91,9% der Theorie, Ethanol wurde mit einer Ausbeute von 75,8% der Theorie und einer Selektivität von 98,9% der Theorie erhalten.

*Beispiel 5:*

*Thermische Spaltung von N-Butylcarbamidsäure-octadecylester*

Zur Spaltung wurde die in Beispiel 2 beschriebene Apparatur benutzt; am oberen Auslass des Dünnschichtverdampfers war ein Schlangenkühler montiert, der während der Spaltung zur Abtrennung des Butylisocyanates von nicht gespaltenem Ausgangsmaterial bzw. Octadecanol diente und dazu mit Wärmeträgeröl auf einer Temperatur von 140° C gehalten wurde. Über eine Destillationsbrücke wurde das Isocyanat in einen auf 150° C gekühlten Schlangenkühler geleitet; das Kondensat wurde in einer auf −20° C gekühlten Vorlage aufgefangen. Über eine mit Aceton/Trockeneis gekühlte Falle war die Spaltapparatur an die Aussenluft angeschlossen.

Während der sechsstündigen Spaltung wurde der geschmolzene N-Butylcarbamidsäureoctadecylester über den auf 120° C thermostatisierten Tropftrichter mit einer Rate von 40 g/h in den mit 380° C heissem Wärmeträgeröl erhitzten Dünnschichtverdampfer kontinuierlich eingespeist, durch den Rührer gleichmässig auf die Innenwandung des Dünnschichters verteilt und während des Herablaufens zu Butylisocyanat und Octadecanol gespalten. Während der Spaltung wurde ein Strom getrockneten Stickstoffs kurz unterhalb des unteren Entnahmebodens mit einer Rate von 300 ml/h in die Apparatur eingeleitet.

Während des Versuches wurden in der Isocyanatvorlage 56 g Butylisocyanat (Reinheit 94,8%) und in der Vorlage am unteren Ende des Dünnschichtverdampfers 182 g eines Gemisches aus 84 Mol-% Octadecanol und 16 Mol-% N-Butylcarbamidsäureoctadecylester aufgefangen.

Diese Werte zeigen hinsichtlich der Bildung von Butylisocyanat eine Ausbeute von 82,5% der Theorie und eine Selektivität von 98,1% der Theorie.

*Beispiel 6:*

*Thermische Spaltung von 2,2-Bis-(4-N-butylcarbamoyloxycyclohexyl)propan*

Zur Durchführung der Spaltung wurde die in Beispiel 2 näher beschriebene Apparatur benutzt; der Schlangenkühler am oberen Auslass des Dünnschichtverdampfers war auf 120° C beheizt.

Während des 4,5stündigen Versuches wurden 300 g 2,2-Bis-(4-N-butylcarbamoyloxycyclohexyl)propan über den auf 160° C thermostatisierten Tropftrichter in den auf 370° C geheizten Dünnschichtverdampfer kontinuierlich eingebracht und dabei zu Butylisocyanat und einem Gemisch aus 2,2-Bis-(4-hydroxycyclohexyl)propan und 2-(4-N-Butylcarbamoyloxycyclohexyl)-2-(4-hydroxycyclohexyl)propan gespalten. Ein Strom trokkenen Stickstoffs wurde kurz unterhalb des unteren Entnahmebodens mit einer Rate von 65 ml/h in die Apparatur eingeleitet.

Bei dieser Spaltung wurden 78 g Butylisocyanat (Reinheit 96,4%) und aus der Vorlage am unteren Ende des Dünnschichtverdampfers 209 g Alkoholfraktion aus 21% 2,2-Bis-(4-hydroxycyclohexyl)propan und 79% 2-(4-N-Butylcarbamoyloxycyclohexyl)-2-(4-hydroxycyclohexyl)propan erhalten.

Daraus errechnet sich für Butylisocyanat eine Ausbeute von 55,5% der Theorie und eine Selektivität von 92,8% der Theorie.

**Patentansprüche**

1. Verfahren zur kontinuierlichen thermischen Spaltung von N-monosubstituierten Carbamidsäureestern, deren Isocyanat- und/oder Hydroxylkomponente bei einem Druck von 0,01 bar einen unterhalb von 250° C liegenden Siedepunkt aufweisen, bei einem Druck von 0,001 bis 20 bar und einer Temperatur von 150 bis 450° C und anschliessender Auftrennung der Spaltprodukte in eine vorwiegend aus der Isocyanatkomponente der Carbamidsäureester bestehende Fraktion A und eine vorwiegend aus der Hydroxylkomponente der Carbamidsäureester bestehende Faktion B, dadurch gekennzeichnet, dass man

a) die thermische Spaltung in einem Rohrreaktor durchführt, wobei die zu spaltenden Carbamidsäureester in flüssiger Form an der Innenwand des Rohrreaktors herablaufen oder an ihr entlanggeführt werden, und

b) die unter den Reaktionsbedingungen gasförmig anfallenden Spaltprodukte am Kopf des Rohrreaktors und die unter den Reaktionsbedingungen

ggf. als Flüssigkeit anfallenden Spaltprodukte am Fuss des Rohrreaktors entnimmt.

2. Verfahren gemäss Ansprüchen 1, dadurch gekennzeichnet, dass man die thermische Spaltung in einem rohrförmigen Dünnschicht- oder Fallfilmverdampfer durchführt.

3. Verfahren gemäss Ansprüchen 1 und 2, dadurch gekennzeichnet, dass man die Spaltprodukte gemeinsam gasförmig am Kopf des Reaktors entnimmt und durch selektive Kondensation in die Fraktionen A und B auftrennt.

4. Verfahren gemäss Ansprüchen 1 und 2, dadurch gekennzeichnet, dass man die Fraktion A gasförmig am Kopf des Reaktors und die Fraktion B flüssig am Fuss des Reaktors entnimmt.

5. Verfahren gemäss Ansprüchen 1 und 2, dadurch gekennzeichnet, dass man die Fraktion A flüssig am Fuss des Reaktors und die Fraktion B gasförmig am Kopf des Reaktors entnimmt.

6. Verfahren gemäss Ansprüchen 1 bis 5, dadurch gekennzeichnet, dass man die thermische Spaltung in Gegenwart von die Spaltungsreaktion beschleunigenden Katalysatoren und/oder die Spaltprodukte stabilisierenden Verbindungen durchführt.

7. Verfahren gemäss Ansprüchen 1 bis 6, dadurch gekennzeichnet, dass man die Spaltung in Anwesenheit geringer Mengen inerter hochsiedender Flüssigkeiten durchführt, mit deren Hilfe unerwünschte, nichtflüchtige Nebenprodukte am Fuss des Reaktors ausgeschleust werden.

8. Verfahren gemäss Ansprüchen 1 bis 7, dadurch gekennzeichnet, dass man die thermische Spaltung in Anwesenheit inerter Trägergase durchführt.

9. Verfahren gemäss Ansprüchen 1 bis 8, dadurch gekennzeichnet, dass man als N-monosubstituierte Carbamidsäureester solche der allgemeinen Formeln

$$R^1(NHCO_2R^2)_x \text{ oder } (R^1NHCO_2)_yR^2$$

einsetzt, wobei

$R^1$ jeweils für einen ggf. inerte Substituenten aufweisenden und/oder olefinisch ungesättigten aliphatischen Kohlenwasserstoffrest mit insgesamt 1 bis 18 Kohlenstoffatomen, einen ggf. inerte Substituenten aufweisenden und/oder olefinisch ungesättigten cycloaliphatischen Kohlenwasserstoffrest mit insgesamt 3 bis 18 Kohlenstoffatomen, einen ggf. inerte Substituenten aufweisenden araliphatischen Kohlenwasserstoffrest mit insgesamt 7 bis 18 Kohlenstoffatomen oder einen ggf. inerte Substituenten aufweisenden aromatischen Kohlenwasserstoffrest mit insgesamt 6 bis 18 Kohlenstoffatomen besteht, wobei es sich bei den Resten $R^1$, den Formeln entsprechend, um x- bzw. einwertige Reste handelt,

$R^2$ jeweils für einen ggf. inerte Substituenten aufweisenden und/oder olefinisch ungesättigten aliphatischen Kohlenwasserstoffrest mit insgesamt 1 bis 18 Kohlenstoffatomen, einen ggf. inerte Substituenten aufweisenden und/oder olefinisch ungesättigten cycloaliphatischen Kohlenwasserstoffrest mit insgesamt 4 bis 18 Kohlenstoffatomen, einen ggf. inerte Substituenten aufweisenden araliphatischen Kohlenwasserstoffrest mit insgesamt 7 bis 18 Kohlenstoffatomen oder einen ggf. inerte Substituenten aufweisenden aromatischen Kohlenwasserstoffrest mit insgesamt 6 bis 12 Kohlenstoffatomen steht, wobei es sich bei den Resten $R^2$, den allgemeinen Formeln entsprechend, um ein- bzw. y-wertige Reste handelt,

x und y für gleiche oder verschiedene, ganze oder (im statistischen Mittel) gebrochene Zahlen von 1 bis 3 steht,

mit der Massgabe, dass solche Carbamidsäureester der genannten allgemeinen Formeln eingesetzt werden, die bei dem im Reaktor vorliegenden Druck einen mindestens 10° C über dem Siedepunkt des höhersiedenden Spaltproduktes liegenden Siedepunkt aufweisen, und deren Isocyanat- und Hydroxylkomponente ihrerseits bei Normaldruck einen mindestens 50° C voneinander verschiedenen Siedepunkt aufweisen.

## Claims

1. Process for the continuous thermal splitting of N-monosubstituted carbamic acid esters the isocyanate and/or hydroxyl component of which have a boiling point below 250° C under a pressure of 0.01 bar, under a pressure of 0.001 to 20 bar and at a temperature of 150 to 450° C, and subsequent separation of the cleavage products into a fraction A, consisting predominantly of the isocyanate component of the carbamic acid esters, and a fraction B, consisting predominantly of the hydroxyl component of the carbamic acid esters, characterised in that

a) the thermal splitting is carried out in a tube reactor, the carbamic acid esters to be split flowing down or being passed along the inner wall of the tube reactor in liquid form, and

b) the cleavage products obtained in gaseous form under the reaction conditions are removed at the top of the tube reactor and those cleavage products possibly obtained in liquid form under the reaction conditions are removed at the bottom of the tube reactor.

2. Process according to Claim 1, characterised in that the thermal splitting is carried out in a tubular thin-layer or falling-film evaporator.

3. Process according to Claims 1 and 2, characterised in that the cleavage products are removed together in gaseous form at the top of the reactor and are separated into fractions A and B by selective condensation.

4. Process according to Claims 1 and 2, characterised in that fraction A is removed in gaseous form at the top of the reactor and fraction B is removed in liquid form at the bottom of the reactor.

5. Process according to Claims 1 and 2, characterised in that fraction A is removed in liquid form at the bottom of the reactor and fraction B is removed in gaseous form at the top of the reactor.

6. Process according to Claims 1 to 5, characterised in that the thermal splitting is carried out in the presence of catalysts which accelerate the splitting reaction and/or compounds which stabilise the cleavage products.

7. Process according to Claims 1 to 6, characterised in that the splitting is carried out in the presence of small quantities of inert high-boiling liquids, with the aid of which undesired, non-volatile by-products are discharged at the bottom of the reactor.

8. Process according to Claims 1 to 7, characterised in that the thermal splitting is carried out in the presence of inert carrier gases.

9. Process according to Claims 1 to 8, characterised in that the N-monosubstituted carbamic acid esters employed are those of the general formulae

$$R^1(NHCO_2R^2)_x \text{ or } (R^1NHCO_2)_yR^2$$

wherein

$R^1$ in each case represents an aliphatic hydrocarbon radical which has a total of 1 to 18 carbon atoms and optionally contains inert substituents and/or is olefinically unsaturated, a cycloaliphatic hydrocarbon radical which has a total of 3 to 18 carbon atoms and optionally contains inert substituents and/or is olefinically unsaturated, an araliphatic hydrocarbon radical which has a total of 7 to 18 carbon atoms and optionally contains inert substituents or an aromatic hydrocarbon radical which has a total of 6 to 18 carbon atoms and optionally contains inert substituents, the radicals $R^1$, as in the formulae, being x- or mono-functional radicals,

$R^2$ in each case represents an aliphatic hydrocarbon radical which has a total of 1 to 18 carbon atoms and optionally contains inert substituents and/or is olefinically unsaturated, a cycloaliphatic hydrocarbon radical which has a total of 4 to 18 carbon atoms and optionally contains inert substituents and/or is olefinically unsaturated, an araliphatic hydrocarbon radical which has a total of 7 to 18 carbon atoms and optionally contains inert substituents, or an aromatic hydrocarbon radical which has a total of 6 to 12 carbon atoms and optionally contains inert substituents, the radicals $R^2$, as in the general formulae, being mono- or y-functional radicals, and

x and y represent identical or different integers or (on a statistical average) fractions from 1 to 3,

with the proviso that those carbamic acid esters of the indicated general formulae are used which have, under the pressure present in the reactor, a boiling point at least 10° C higher than the boiling point of the higher-boiling cleavage product, and the isocyanate and hydroxyl components of which have, in their turn, boiling points which differ from each other by at least 50° C under normal pressure.

## Revendications

1. Procédé pour la dissociation thermique continue d'esters d'acides carbamiques N-monosubstitués dont le composant isocyanate et/ou le composant hydroxy ont, sous une pression de 0,01 bar, un point d'ébullition inférieur à 250° C, cette dissociation étant effectuée sous une pression de 0,001-20 bar et à une température de 150 à 450° C, avec séparation ultérieure des produits de dissociation en une fraction A constituée principalement du composant isocyanate des esters d'acides carbamiques, ainsi qu'en une fraction B constituée principalement du composant hydroxy des esters d'acides carbamiques, caractérisé en ce que:

a) on effectue la dissociation thermique dans un réacteur tubulaire, les esters d'acides carbamiques à dissocier descendant sous forme liquide sur la paroi intérieure du réacteur tubulaire ou s'écoulant le long de cette dernière, et

b) on retire les produits de dissociation se formant à l'état gazeux dans les conditions réactionnelles en tête du réacteur tubulaire et l'on retire les produits de dissociation se formant éventuellement sous forme d'un liquide dans les conditions réactionnelles au pied du réacteur tubulaire.

2. Procédé selon la revendication 1, caractérisé en ce qu'on effectue la dissociation thermique dans un évaporateur tubulaire à couche mince ou à pellicule descendante.

3. Procédé selon les revendications 1 et 2, caractérisé en ce qu'on retire les produits de dissociation ensemble à l'état gazeux en tête du réacteur et on les sépare en fractions A et B par condensation sélective.

4. Procédé selon les revendications 1 et 2, caractérisé en ce qu'on retire la fraction A à l'état gazeux en tête du réacteur et la fraction B à l'état liquide au pied de ce dernier.

5. Procédé selon les revendications 1 et 2, caractérisé en ce qu'on retire la fraction A à l'état liquide au pied du réacteur et la fraction B à l'état gazeux en tête de ce dernier.

6. Procédé selon les revendications 1 à 5, caractérisé en ce qu'on effectue la dissociation thermique en présence de catalyseurs accélérant la réaction de dissociation et/ou de composés stabilisant les produits de dissocation.

7. Procédé selon les revendications 1 à 6, caractérisé en ce qu'on effectue la dissociation en présence de faibles quantités de liquides inertes à point d'ébullition élevé à l'aide desquels on expulse les sous-produits non volatils et non désirés au pied du réacteur.

8. Procédé selon les revendications 1 à 7, caractérisé en ce qu'on effectue la dissociation thermique en présence de gaz supports inertes.

9. Procédé selon les revendications 1 à 8, caractérisé en ce que, comme esters d'acides carbamiques N-monosubstitués, on utilise ceux répondant aux formules générales:

$$R^1(NHCO_2R^2)_x \text{ ou } (R^1NHCO_2)_yR^2$$

où

$R^1$ représente chaque fois un radical d'hydrocarbure aliphatique comportant éventuellement des substituants inertes et/ou à insaturation oléfinique et contenant, au total, 1 à 18 atomes de carbone, un radical d'hydrocarbure cycloaliphatique comportant éventuellement des substituants inertes et/ou à insaturation oléfinique et contenant, au total, 3 à 18 atomes de carbone, un radical d'hydrocarbure araliphatique comportant éventuellement des substituants inertes et contenant, au total, 7 à 18 atomes de carbone ou un radical d'hydrocarbure aromatique comportant éventuellement des substituants inertes et contenant, au total, 6 à 18 atomes de carbone et, dans le cas des radicaux $R^1$, conformément aux formules, il s'agit de radicaux x-valents ou monovalents,

$R^2$ représente chaque fois un radical d'hydrocarbure aliphatique comportant éventuellement des substituants inertes et/ou à insaturation oléfinique et contenant, au total, 1 à 18 atomes de carbone, un radical d'hydrocarbure cycloaliphatique comportant éventuellement des substituants inertes et/ou à insaturation oléfinique et contenant, au total, 4 à 18 atomes de carbone, un radical d'hydrocarbure araliphatique comportant éventuellement des substituants inertes et contenant, au total, 7 à 18 atomes de carbone, ou un radical d'hydrocarbure aromatique comportant éventuellement des substituants inertes et contenant, au total, 6 à 12 atomes de carbone et, dans le cas des radicaux $R^2$, conformément aux formules générales, il s'agit de radicaux monovalents ou y-valents,

x et y sont des nombres entiers ou (en moyenne statistique) fractionnaires de 1 à 3, identiques ou différents,

avec cette réserve que l'on utilise les esters d'acides carbamiques des formules générales mentionnées qui, sous la pression régnant dans le réacteur, ont un point d'ébullition supérieur d'au moins 10° C à celui du produit de dissociation à point d'ébullition supérieur, les composants isocyanate et hydroxy de ces esters ayant, à leur tour, des points d'ébullition différents d'au moins 50° C sous pression normale.